(19) Europäisches Patentamt
European Patent Office
Office européen des brevets

(11) Publication number: **0 178 076**
**B1**

(12) # EUROPEAN PATENT SPECIFICATION

(45) Date of publication of patent specification: **02.05.90**

(51) Int. Cl.$^5$: **C 07 F 7/08**, C 07 F 7/18, A 61 K 31/695

(21) Application number: **85306413.7**

(22) Date of filing: **10.09.85**

(54) **Organo-silicone compounds, process for their manufacture, and pharmaceutical compositions thereof.**

(30) Priority: **10.09.84 JP 188197/84**

(43) Date of publication of application:
**16.04.86 Bulletin 86/16**

(45) Publication of the grant of the patent:
**02.05.90 Bulletin 90/18**

(84) Designated Contracting States:
**CH DE FR GB IT LI NL SE**

(56) References cited:
**EP-A-0 046 242**

**CHEMICAL ABSTRACTS, vol. 101, no. 17, 22nd October 1984, page 736, abstract no. 152067u, Columbus, Ohio, US; & JP-A-59 98 091 (SANWA KAGAKU KENKYUSHO CO., LTD) 06-06-1984**

**CHEMICAL ABSTRACTS, vol. 95, no. 17, 26th October 1981, page 676, abstract no. 150701e, Columbus, Ohio, US; & JP-A-81 63 966 (TOKYO KINZOKU KOGYO CO., LTD) 30-05-1981**

(73) Proprietor: **SANWA KAGAKU KENKYUSHO CO., LTD.**
**No. 35, Higashi-sotobori-cho**
**Higashi-ku Nagoya-shi Aichi-ken (JP)**

(73) Proprietor: **Shin-Etsu Chemical Co., Ltd.**
**6-1, Ohtemachi 2-chome**
**Chiyoda-ku Tokyo 100 (JP)**

(72) Inventor: **Toyoshima, Shigeshi**
**3-14-22, Funabashi**
**Setagaya-ku Tokyo (JP)**
Inventor: **Kurono, Masayasu**
**1-7-17, Kakeage Minami-ku**
**Nagoya-shi Aichi-ken (JP)**
Inventor: **Unno, Ryoichi**
**1-3, Hikarigaoka Chikusa-ku**
**Nagoya-shi Aichi-ken (JP)**
Inventor: **Kimura, Hiromoto**
**4-130, Jojo-cho**
**Kasugai-shi Aichi-ken (JP)**
Inventor: **Ito, Koichi**
**Shin-Etsu Chemical Co. Ltd. 6-1, Ohtemachi 2-chome**
**Chiyoda-ku Tokyo (JP)**

**EP 0 178 076 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(72) Inventor: Ishihara, Toshinobu Shin-Etsu Chemical Co., Ltd.
Gosei-gijutsu Kenkyusho 28-1 Ohaza-Nishi-fukushima
Kubiki-mura Nak-kubiki-gun Niigata-ken (JP)
Inventor: Yamamoto, Akira Shin-Etsu Chemical Co., Ltd.
Gosei-gijutsu Kenkyusho 28-1 Ohaza-Nishi-fukushima
Kubiki-mura Naka-kubiki-gun Niigata-ken (JP)

(74) Representative: Diamond, Bryan Clive et al
Gee & Co., Chancery House, Chancery Lane
London WC2A 1QU (GB)

**Description**

The present invention relates to novel organo-silicone compounds, a process for their manufacturè, and to anti-tumour pharmaceutical compositions comprise as an effective component, at least one of the compounds.

As silicon-containing compounds having a pharmaceutical action against cancers or other tumours, so-called "Silatorane Compounds" have been known, but these compounds have a disadvantage in actual or clinical use, in view of their high toxicity.

There is a compound of 5-fluorouracil which has actually and clinically been employed as an anti-tumor agent. This compound has an excellent anti-tumor action but shows in oral dosage a high toxicity in that it often causes trouble in the digestive tract and thus this compound has exclusively been dosed by injection. In order to overcome this disadvantage, namely to make oral dosage possible, 1-(2-tetrahydrofuryl)-5-fluorouracil has been used; this compound shows a lower toxicity in oral dosage but has a disadvantage in that its anti-tumor activity is low.

Meanwhile, in Jap. Unexamined Pat. Applns Gazette Nos. 63 966/1981 and 38 789/1982, there are disclosed 1-[2-(2-trimethylsilylethyl)thio]ethylcarbamoyl]-5-fluorouracil and 2-[2-(aminoethylthio)]ethyltrimethylsilane, respectively. These compounds have a higher anti-tumor activity but there are some doubts in actual use thereof, since each has a high, toxicity similar to the Silatorane compounds and shows an undesirable side-reaction.

An object of the invention is to provide novel organo-silicone compounds, each having a high anti-tumor activity and having no or little toxicity, particularly in oral dosage, whereby the conflict between level of anti-tumor activity and safety in actual use, which has been encountered in the prior anti-tumor agents, can be overcome.

The invention provides novel organo-silicone compounds represented by the general formula:

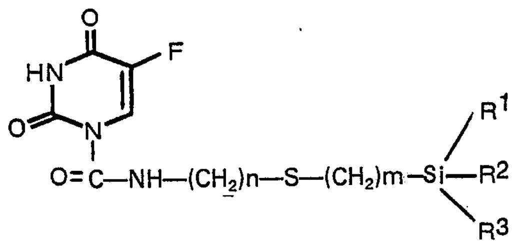

wherein $R^1$, $R^2$ and $R^3$ are each an alkyl group of 1 to 10 carbon atoms or a phenyl radical, and *m* and *n* are each an integer of 2 or 3, but are not both 2 when each of $R^1$, $R^2$ and $R^3$ represents a methyl radical.

Each of the compounds of Formula 1 exhibits an excellent anti-tumor activity and a quite low toxicity.

The alkyl group may be a straight-chain alkyl radical, branched-chain alkyl radical. As examples for the straight-chain alkyl radicals, methyl, ethyl, n-propyl, n-butyl, n-pentyl, n-hexyl, n-decyl and the like may be listed. As examples for the branched-chain alkyl radicals, isopropyl, isobutyl, s-butyl, t-butyl, isopentyl and the like radicals may be listed.

According to the method of the invention, the compound shown by Formula I can be prepared by reacting a compound represented by the formula:

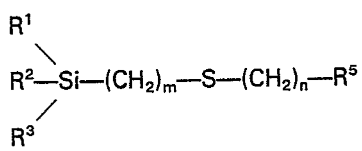

(II)

wherein $R^1$, $R^2$, $R^3$, *m* and *n* have the meaning referred to, and $R^5$ is a radical $-N{=}C{=}O$, $-NH_4$ or $-N_4-CO-Cl$, with a compound represented by the formula,

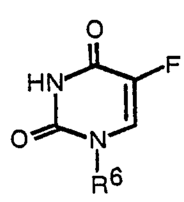

(III)

wherein $R^6$ is hydrogen, alkali metal or a radical of $-COCl$.

In particular embodiments, the compound shown by Formula I can be prepared in accordance with one of the following methods.

(A) A method, in which a compound represented by the formula,

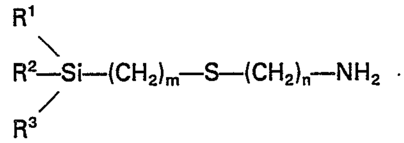

(IIa)

wherein $R^1$, $R^2$, $R^3$, *m* and *n* have the meaning referred to, is reacted with a 1-chloroformyl-5-fluorouracil represented by the formula,

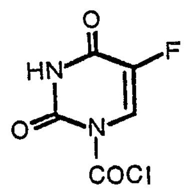

(III–a)

This reaction proceeds by merely stirring both raw materials in an equimolar amount, in the presence of a solvent. As the solvent, pyridine, N,N-dimethylformamide, N,N-dimethylacetamide, dimethylsulfoxide, hexamethylphosphoric triamide, acetonitrile, nitromethane and the like may be used. A reaction temperature depends on kinds of the raw materials and solvent and thus it is not always the same but a temperature in a range of 0 to 10°C is preferable, since said chloroformyl compound shown by the Formula III-a is, in general, not so stable.

The starting material of 1-chloroformyl-5-fluorouracil can be synthesized according to the method as disclosed in "Bull. Chem. Soc. Japan", Vol. 50, No. 9, Pages 2406 to 2412 (1977).

(B) A method, in which a compound represented by the formula

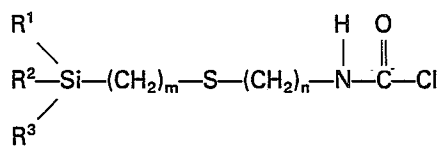

(II-b)

wherein $R^1$, $R^2$, $R^3$, *m* and *n* have the meaning referred to, is reacted with 5-substituted uracil represented by the formula,

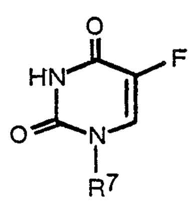

(III–b)

wherein $R^7$ is hydrogen or an alkali metal.

This reaction also proceeds by merely stirring both raw materials in an equimolar amount, in the presence of a solvent. As the solvent, those referred to in the Method (A) may be employed. The reaction temperature depends on kinds of the raw materials and solvent but, in general, 0 to 50°C is preferable.

The raw material shown by the Formula II-b can be prepared by reacting the compound shown by the Formula II-a with phosgene in an equimolar amount. In general, the reaction can conveniently proceed by using a base in the presence of a solvent. As the base, triethylamine, pyridine, potassium carbonate or the like may be used. As the solvent, carbon tetrachloride or the like halogenated carbons, ethyl ether, tetrahydrofuran, dioxane or the like ethers, benzene, toluene, xylene or the like aromatic hydrocarbons may be employed. The reaction temperature depends on kinds of the raw material and solvent and thus it is not always the same but, in general, a temperature of 0 to 20°C is selected. The resulting carbamoylchloride shown by the Formula II-b can be used for the reaction with the compound shown by the Formula III-b, without any purifying procedure.

(C) The compounds shown by the Formula I, namely those represented by the formula,

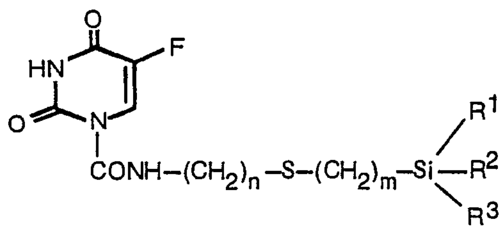

(I–a)

wherein $R^1$, $R^2$, $R^3$, *m* and *n* have the meaning referred to, can also be prepared by reacting an isocyanate represented by the formula,

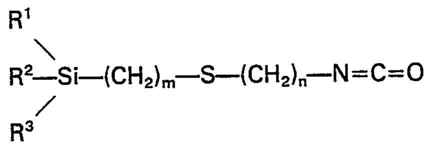

(II-c)

wherein $R^1$, $R^2$, $R^3$, *m* and *n* have the meaning referred to, with 5-fluorouracil represented by the formula,

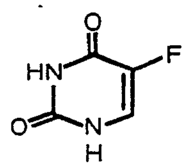

(III–c)

This reaction also proceeds by merely stirring both raw materials in an equimolar amount, in the presence of a solvent. As the solvent, those referred to on the Method (A) can be employed. The reaction temperature depends on kinds of the raw materials and solvent and thus it is not always in a constant level but, in general, a temperature of 0 to 100°C is selected.

The raw material shown by the Formula II-c can be prepared by reacting the compound shown by the Formula II-a with phosgene in 1.0 to 3.0 times molar amount. This reaction, in general, proceeds with by merely stirring the reactants in the presence of a solvent. As the solvent, benzene, toluene, xylene or the like aromatic hydrocarbon can be employed. The reaction temperature depends on kinds of the raw material and solvent but a temperature of 0°C to a boiling point of the solvent is usually selected.

An organo-silicone compound according to the invention has an anti-tumor activity and more particularly an inhibiting action to various solid tumors and shows no or quite low toxicity in both oral and non-oral dosage thereof. Therefore, it can be used as a safety effective component for anti-tumor agents.

Each of the compounds according to the invention can be dosed in both oral and non-oral routes. For the oral dosage, the compound may be made with conventional additives or carriers into a tablet, capsule, granule or other form. For the non-oral dosage, the compound may be made with conventional additives or carriers into an injection, suppository or the like form.

A dosing amount of the compound for human depends on kind of the compound to be used, condition of illness, age of a patient, form of the medicine and other factors but for an adult, 300 to 5000 mg/day of oral dosage and 100 to 2000 mg/day by suppository are preferable.

The invention will now be explained with reference to Examples and Pharmaceutical Test Examples.

Example 1

5-Fluoro-3,4-dihydro-2,4-dioxo-N-[2-[[3-(trimethylsilyl)propyl]-thio]ethyl]-1(2H)-pyrimidinecarboxamide

Trichloromethyl chloroformate (9.90 g, 50.0 mmol) was added dropwise with stirring to a mixture of 5-fluoro-2,4(1H,3H)-pyrimidinedione (13.0 g, 0.100 mol) and active carbon (3.0 g) in 400 ml of dry pyridine, while the temperature was maintained at 5°C.

After stirring for one hour, the unreacted phosgene was removed in vacuo at 5°C.

Then, to the reaction mixture 2-[[3-(trimethylsilyl)propyl]thio]ethyl amine (9.57 g, 50.0 mmol) was added dropwise at 10°C under argon atmosphere, and stirred for one hour. The reaction mixture was evaporated to dryness in vacuo, the resulting residue was dissolved in ethyl acetate (400 ml) and then the active carbon was filtered off, the filtrate was washed with 3% hydrochloric acid (300 ml) and then with water (500 ml). The organic phase was dried over sodium sulfate and the solvent was evaporated to dryness in vacuo.

The crude crystals were recrystallized from ethyl ether to yield the desired compound (15.7 g, 90.7%).

Melting point: 109—110°C

Elementary analysis: $C_{13}H_{22}FN_3O_3SSi$

Cal.: C, 44.94; H, 6.38; N, 12.09

Found: C, 44.75; H, 6.47; N, 11.99

IR ($\nu_{max}^{KBr}$) $cm^{-1}$:

3300 ($\nu_{NH}$), 2960, 2920 ($\nu_{CH}$), 1740, 1700 ($\nu_{C=O}$), 1525 ($\delta_{NH}$), 1250 ($\nu_{C-Si}$)

$^1$H—NMR ($CDCl_3$) δ ppm:

0.03 (9H, s, —$Si(CH_3)_3$)
0.83 (2H, $A_2B_2$, ⩾$SiCH_2$—)
1.60 (2H, m, ⩾$SiCH_2CH_2CH_2S$—)
2.58 (2H, t, J=7.6Hz, ⩾$SiCH_2CH_2CH_2S$—)
2.74 (2H, t, J=6.4Hz, —$SCH_2CH_2NH$—)
3.61 (2H, q, J=6.4Hz, —$SCH_2CH_2NH$—)
7.44 (1H, d, J=7.0Hz, $C_6$—H)
9.00—9.60 (2H, m, $N_3$—H, $N_8$—H)

MS (EI/DI) m/z:

347 ($M^+$), 332, 202, 73 (base peak).

## Example 2

5-Fluoro-3,4-dihydro-2,4-dioxo-N-[2-[[(2-triethylsilyl)ethyl]thio]ethyl]-1(2H)-pyrimidinecarboxamide

This compound was prepared by the similar procedure as in the case of Example 1, except for the treatment with 2-[[2-(triethylsilyl)ethyl]thio]ethyl amine (11.0 g, 50.0 mmol) instead of 2-[[3-(trimethylsilyl)propyl]thio]ethyl amine. The crude material was recrystallized from ethyl ether to yield the desired compound (17.5 g, 93.2%).

Melting point: 76°C

Elementary analysis: $C_{15}H_{26}FN_3O_3SSi$

Cal.: C, 47,97; H, 6.98; N, 11.19

Found: C, 47.67; H, 7.22; N, 11.17

IR ($\nu_{max}^{KBr}$) $cm^{-1}$:

3320 ($\nu_{NH}$), 2950($\nu_{CH}$), 1740, 1700 ($\nu_{C=O}$), 1525 ($\delta_{NH}$), 1230 ($\nu_{C-Si}$)

$^1$H—NMR ($CDCl_3$) δ ppm:

0.27—1.36 (17H, m, —$CH_2Si(C_2H_5)_3$)
2.54 (2H, $A_2B_2$, —>$SiCH_2CH_2S$—)
2.74 (2H, t, J=7.0Hz, —$SCH_2CH_2NH$—)
3.58 (2H, q, J=7.0Hz, —$SCH_2CH_2NH$—)
8.23 (1H, m, $N_3$—H)
8.42 (1H, d, J=7.0Hz, $C_6$—H)
9.23 (1H, m, $N_8$—H)

MS (EI/DI) m/z:

216, 188 (base peak)

## Example 3

N-[2-[[2-(Dimethylphenylsilyl)ethyl]thio]ethyl]-5-fluoro-3,4-dihydro-2,4-dioxo-1(2H)-pyrimidinecarboxamide

This compound was prepared by the similar procedure as in the case of Example 1, except for treatment with 2-[[2-(dimethylphenylsilyl)ethyl]thio]ethyl amine (10.8 g, 45.0 mmol) instead of 2-[[3-trimethylsilyl)propyl]thio]ethyl amine. The crude material was recrystallized from ethyl ether to yield the desired compound (15.5 g, 87.3%).

Melting point: 90—91°C

Elementary analysis: $C_{15}H_{26}FN_3O_3SSi$

Cal.: C, 51.62; H, 5.61; N, 10.62

Found: C, 51.66; H, 5.71; N, 10.54

IR ($\nu_{max}^{KBr}$) $cm^{-1}$:

3350, 3310 ($\nu_{NH}$), 2960, 2930 ($\nu_{CH}$), 1735 ($\nu_{C=O}$), 1505 ($\delta_{NH}$)

$^1$H—NMR ($CDCl_3$) δ ppm:

0.27 (6H, s, —$Si(CH_3)_2Ph$)
1.10 (2H, $A_2B_2$, ⩾Si—$CH_2$—)
2.49 (2H, $A_2B_2$, ⩾$SiCH_2CH_2S$—)
2.69 (2H, t, J=6.0Hz, —$SCH_2CH_2N$—)
3.52 (2H, q, J=6.0Hz, —$SCH_2CH_2N$—)
7.33 (5H, m, —$S(CH_3)_2Ph$)
8.40 (1H, d, J=7.0Hz, $C_6$—H)
9.25 (2H, m, $N_3$—H, $N_8$—H)

FD—MS m/z:

395 ($M^+$, base peak)

Example 4

N-[2-[[2-(Dimethyloctylsilyl)ethyl]thio]ethyl]-5-fluoro-3,4-dihydro-2,4-dioxo-1(2H)-pyrimidinecarboxamide

This compound was prepared by the similar procedure as in the case of Example 1, except for treatment with 2-[[2-(dimethyloctylsilyl)ethyl]thio]ethyl amine (13.8 g, 50.0 mmol) instead of 2-[[3-(trimethylsilyl]propyl]thio]ethyl amine. The crude material was recrystallized from ethyl ether-n-hexane to yield the desired compound (17.1 g, 79.1%).

Melting point: 60—61°C

Elementary analysis: $C_{19}H_{31}FN_3O_3SSi$

Cal.: C, 52.87; H, 7.94; N, 9.74

Found: C, 52.63; H, 8.26; N, 9.71

IR ($\nu_{max}^{KBr}$) $cm^{-1}$:

3430 ($\nu_{NH}$), 2930, 2860 ($\nu_{CH}$), 1735 ($\nu_{C=O}$), 1530 ($\delta_{NH}$)

$^1H$—NMR ($CDCl_3$) δ ppm:

— 0.05 (6H, s, —$Si(CH_3)_2$)

0.22—1.52 (19H, m, —$CH_2Si(CH_2)_7CH_3$)

2.46 (2H, $A_2B_2$, ⩾$SiCH_2CH_2S$—)

2.72 (2H, t, J=6.0Hz, —$SH_2CH_2NH$—)

3.57 (2H, q, J=6.0Hz, —$SCH_2CH_2NH$—)

8.42 (1H, d, J=7.0Hz, $C_6$—H)

9.27 (2H, m, $N_3$—H, $N_8$—H)

MS (EI/DI) m/z:

188, 160 (base peak)

Example 5

5-Fluoro-3,4-dihydro-N-[2-[[2-(isopropyldimethylsilyl)ethyl]thio]ethyl]-2,4-dioxo-1(2H)-pyrimidinecarboxamide

This compound was prepared by the similar procedure as in the case of Example 1, except for treatment with 2-[[2-(isopropyldimethylsilyl)ethyl]thio]ethyl amine (10.3 g, 50.0 mmol) instead of 2-[[3-(trimethylsilyl)propyl]thio]ethyl amine. The crude material was recrystallized from ethyl ether-n-hexane to yield the desired compound (16.8 g, 93.0%).

Melting point: 85—86°C

Elementary analysis: $C_{14}H_{24}FN_3O_3SSi$

Cal.: C, 46.51; H, 6.69; N, 11.62

Found: C, 46.34; H, 6.87; N, 11.63

IR ($\nu_{max}^{KBr}$) $cm^{-1}$:

3420 ($\nu_{NH}$), 2960, ($\nu_{CH}$), 1725 1690 ($\nu_{C=O}$), 1525 ($\delta_{NH}$), 1250 ($\nu_{C-Si}$)

$^1H$—NMR ($CDCl_3$) δ ppm:

— 0.06 (6H, s, —$Si(CH_3)_2$)

0.32—1.15 (9H, m, —$CH_2SiCH(CH_3)_2$)

2.12—3.09 (4H, m, ⩾$SiCH_2CH_2SCH_2$—)

3.57 (2H, q, J=6.0Hz, —$SCH_2CH_2NH$—)

8.42 (1H, d, J=7.0Hz, $C_6$—H)

9.30 (2H, m, $N_3$—H, $N_8$—H)

FD—MS m/z:

361($M^+$), 318 (base peak)

Example 6

N-[2-[[2-(Butyldimethylsilyl)ethyl]thio]ethyl]-5-fluoro-3,4-dihydro-2,4-dioxo-1(2H)-pyrimidinecarboxamide

This compound was prepared by the similar procedure as in the case of Example 1, except for treatment with 2-[[2-(butyldimethylsilyl)ethyl]thio]ethyl amine (11.0 g, 50.0 mmol) instead of 2-[[3-

(trimethylsilyl)propyl]thio]ethyl amine. The crude material was recrystallized from methanol to yield the desired compound (17.2 g, 91.5%).

Melting point: 71—72°C

Elementary analysis: $C_{15}H_{26}FN_3O_3SSi$

Cal.: C, 47.98; H, 6.98; N, 11.19

Found: C, 47.63; H, 7.14; N, 11.14

IR ($\nu_{max}^{KBr}$) $cm^{-1}$:

3300 ($\nu_{NH}$), 2960, 2920 ($\nu_{CH}$), 1735 ($\nu_{C=O}$), 1520 ($\delta_{NH}$), 1250 ($\nu_{C-Si}$)

$^1H$—NMR ($CDCl_3$) δ ppm:

— 0.04 (6H, s, —$Si(CH_3)_2$)

0.16—1.62 (11H, m. —$CH_2Si(CH_2)_3CH_3$)

2.26—3.19 (4H, m, ⩾$SiCH_2CH_2SCH_2$—)

3.39—3.82 (2H, m, —$SCH_2C_2CH_2NH$—)

8.42 (1H, d, J=6.0Hz, $C_6$—H)

9.21 (2H, m, $N_3$—H, $N_8$—H)

FAB—MS m/z:

376 [$(M+1)^+$]

Example 7

5-Fluoro-3,4-dihydro-N-[2-[[2-(methyldiphenylsilyl)ethyl]thio]ethyl]-2,4-dioxo-1(2H)-pyrimidinecarboxamide

This compound was prepared by the similar procedure as in the case of Example 1, except for treatment with 2-[[2-(methyldiphenylsilyl)ethyl]thio]ethyl amine (12.0 g, 40.0 mmol) instead of 2-[[3-(trimethylsilyl)propyl]thio]ethyl amine. The crude material was recrystallized from methanol to yield the desired compound (12.6 g, 69.0%).

Melting point: 119—120°C

Elementary analysis: $C_{22}H_{24}FN_3O_3SSi$

Cal.: C, 57.75; H, 5.29; N, 9.18

Found: C, 57.51; H, 5.32; N, 9.06

IR ($\nu_{max}^{KBr}$) $cm^{-1}$:

3430 ($\nu_{NH}$), 2840, ($\nu_{CH}$), 1740 ($\nu_{C=O}$), 1520 ($\delta_{NH}$), 1235 ($\nu_{C-Si}$)

$^1H$—NMR ($CDCl_3$) δ ppm:

0.50 (3H, s, $Ph_2CH_3Si$—)

1.34 (2H, $A_2B_2$, ⩾$SiCH_2$—)

2.50 (2H, $A_2B_2$, ⩾$SiCH_2CH_2Si$—)

2.63 (2H, t, J=6.0z, —$SCH_2CH_2NH$—)

3.45 (2H, q, J=6.0Hz, —$SCH_2CH_2NH$—)

7.28 (10H, m, $Ph_2CH_3Si$—)

8.34 (1H, d, J=6.0Hz, $C_6$—H)

9.17 (2H, m, $N_3$—H, $N_8$—H)

MS (EI/DI) m/z:

327, 285, 197 (base peak)

Example 8

5-Fluoro-3,4-dihydro-2,4-dioxo-N-[3-[[2-(trimethylsilyl)ethyl]thio]propl]-1-(2H)-pyrimidinecarboxamide

This compound was prepared by the similar procedure as in the case of Example 1, except for treatment with 3-[[2-(trimethylsilyl)ethyl]thio]propyl amine (9.55 g, 50.0 mmol) instead of 2-[[3-(trimethylsilyl)propyl]thio]ethyl amine. The crude material was recrystallized from ethyl ether-n-hexane to yield the desired compound (13.3 g, 76.4%).

Melting point: 114—115°C

Elementary analysis: $C_{13}H_{22}FN_3O_3SSi$

Cal.: C, 44.94; H, 6.38; N, 12.09

Found: C, 44.87; H, 6.55; N, 12.11

IR ($\nu_{max}^{KBr}$) $cm^{-1}$:

3330 ($\nu_{NH}$), 2960, ($\nu_{CH}$), 1740 ($\nu_{C=O}$), 1505 ($\delta_{NH}$), 1250 ($\nu_{C-Si}$)

$^1H$—NMR ($CDCl_3$) δ ppm:

0.03 (9H, s, $(CH_3)_3Si$—)

0.81 (2H, $A_2B_2$, ⩾$SiCH_2$—)

1.87 (2H, m, —$SCH_2CH_2CH_2NH$—)

2.21—2.94 (4H, m, ⩾$SiCH_2CH_2SCH_2$—)

3.49 (2H, q, J=6.0Hz, $CH_2NH$—)

8.42 (1H, d, J=7.0Hz, $C_6$—H)

9.10 (1H, m, $N_3$—H)

9.50 (1H, m, $N_8$—H)

MS (EI/DI) m/z:

347($M^+$), 305, 73 (base peak)

Example 9

5-Fluoro-3,4-dihydro--N-[3-[[2-(isopropyldimethylsilyl)ethyl]thio]propl]-2,4-dioxo-1-(2H)-pyrimidinecarboxamide

This compound was prepared by the similar procedure as in the case of Example 1, except for treatment with 3-[[2-(isopropyldimethylsilyl)ethyl]thio]propyl]thio]ethyl amine. The crude material was recrystallized from ethyl ether to yield the desired compound (17.3 g, 92.0%).

Melting point: 122—123°C

Elementary analysis: $C_{15}H_{26}FN_3O_3SSi$

| | | | |
|---|---|---|---|
| Cal.: | C, 47.98; | H, 6.98; | N, 11.19 |
| Found: | C, 48.16; | H, 7.10; | N, 11.40 |

IR ($\nu_{max}^{KBr}$) $cm^{-1}$:

3330 ($\nu_{NH}$), 2950, ($\nu_{CH}$), 1735, 1660 ($\nu_{C=O}$), 1250 ($\nu_{C-Si}$)

$^1$H—NMR ($CDCl_3$) δ ppm:

— 0.03 (6H, s, —Si($CH_3$)$_2$)
0.58—1.11 (9H, m, —$CH_2SiCH(CH_3)_2$)
1.89 (2H, q, J=7.0Hz, —$SCH_2CH_2CH_2NH$—)
2.35—2.78 (4H, m, —$CH_2SCH_2$—)
3.53 (2H, q, J=6.5Hz, —$CH_2NH$—)
8.46 (1H, d, J=7.0Hz, $C_6$—H)
9.05 (2H, m, $N_3$—H, $N_8$—H)

FD—MS m/z:

375($M^+$), 332 (base peak)

Example 10

5-Fluoro-3,4-dihydro-2,4-dioxo-N-[3-[[3-(trimethylsilyl)propyl]thio]propyl]-1(2H)-pyrimidinecarboxamide

This compound was prepared by the similar procedure as in the case of Example 1, except for treatment with 3-[[3-(trimethylsilyl)propyl]thio]propyl amine (10.3 g, 50.0 mmol) instead of 2-[[3-(trimethyl-silyl)propyl]thio]ethyl amine. The crude material was recrystallized from ethyl ether-n-hexane to yield the desired compound (16.6 g, 92.0%).

Melting point: 105—106°C

Elementary analysis: $C_{14}H_{24}FN_3O_3SSi$

| | | | |
|---|---|---|---|
| Cal.: | C, 46.51; | H, 6.69; | N, 11.62 |
| Found: | C, 46.80; | H, 6.71; | N, 11.74 |

IR ($\nu_{max}^{KBr}$) $cm^{-1}$:

3330 ($\nu_{NH}$), 2960, ($\nu_{CH}$), 1735, 1670 ($\nu_{C=O}$), 1250 ($\nu_{C-Si}$)

$^1$H—NMR ($CDCl_3$) δ ppm:

— 0.02 (9H, s, $(CH_3)_3Si$—)
0.42—0.75 (2H, m, ⩾$SiCH_2$—)
1.22—2.15 (4H, m, —$CH_2CH_2SCH_2CH_2CH_2NH$—)
2.32—2.75 (4H, m, —$CH_2SCH_2$—)
3.52 (2H, q, J=6.5Hz, —$CH_2NH$—)
8.45 (1H, d, J=7.0Hz, $C_6$—H)
9.05 (1H, m, $N_3$—H)
9.45 (1H, m, $N_8$—H)

FD—MS m/z:

361($M^+$), 73 (base peak)

Example 11

5-Fluoro-3,4-dihydro-N-[3-[[3-(isopropyldimethylsilyl)propyl]thio]propyl]-2,4-dioxo-1(2H)-pyrimidinecarboxamide

This compound was prepared by the similar procedure as in the case of Example 1, except for treatment with 3-[[3-(isopropyldimethylsilyl)propyl]thio]propyl amine (11.7 g, 50.0 mmol) instead of 2-[[3-

(trimethylsilyl)propyl]thio]ethyl amine. The crude material was recrystallized from ethyl ether-n-hexane to yield the desired compound (16.4 g, 84.3%).

Melting point: 101—102°C

Elementary analysis: $C_{16}H_{28}FN_3O_3SSi$

Cal.: C, 49.33; H, 7.24; N, 10.79

Found: C, 49.52; H, 7.10; N, 10.60

IR ($\nu_{max}^{KBr}$) $cm^{-1}$:

3330 ($\nu_{NH}$), 2950 ($\nu_{CH}$), 1720 ($\nu_{C=O}$), 1670 ($\nu_{CONH}$)

$^1H$—NMR ($CDCl_3$) δ ppm:

— 0.03 (6H, s, $(CH_3)_2Si$—)

0.19—1.15 (8H, m, $(CH_3)_2$—CH—Si—$CH_2$—)

1.25—2.25 (5H, m, $(CH_3)_2CH$—$SiCH_2CH_2CH_2$—$SCH_2CH_2$—)

2.35—2.75 (4H, m, —$CH_2SCH_2$—)

3.54 (2H, q, J=6.0Hz, —$NHCH_2$—)

8.46 (1H, d, J=7.0Hz, $C_6$—H)

9.05 (2H, br., 2 × NH)

FD/MS m/z:

346 (base peak)

## Example 12

5-Fluoro-3,4-dihydro-N-[2-[[3-(isopropyldimethylsilyl)propyl]thio]ethyl]-2,4-dioxo-1(2H)-pyrimidinecarboxamide

This compound was prepared by the similar procedure as in the case of Example 1, except for treatment with 2-[[3-(isopropyldimethylsilyl)propyl]thio]ethyl amine (11.0 g, 50.0 mmol) instead of 2-[[3-(trimethylsilyl)propyl]thio]ethyl amine. The crude material was recrystallized from ethyl to yield the desired compound (16.7 g, 89.0%).

Melting point: 113—114°C

Elementary analysis: $C_{15}H_{26}FN_3O_3SSi$

Cal.: C, 47.98; H, 6.98; N, 11.19

Found: C, 48.15; H, 7.11; N, 11.00

IR ($\nu_{max}^{KBr}$) $cm^{-1}$:

3330 ($\nu_{NH}$), 2950 ($\nu_{CH}$), 1750 ($\nu_{C=O}$), 1680 ($\nu_{CONH}$)

$^1H$—NMR ($CDCl_3$) δ ppm:

— 0.02 (6H, s $(CH_3)_2Si$—)

0.40—1.16 (8H, m, $(CH_3)_2CH$—Si—$CH_2$—)

1.32—1.92 (3H, m, $(Ch_3)_2CH$— $SiCH_2CH_2$—)

2.45—2.92 (4H, m, —$CH_2SCH_2$—)

3.63 (2H, q, J=6.5Hz, —$NHCH_2$—)

8.45 (1H, d, J=7.2Hz, $C_6$—H)

9.22 (2H, br., 2 × NH)

## Example 13

N-[2-[[3-(t-Butyldimethylsilyl)propyl]thio]ethyl]-5-fluoro-3,4-dihydro-2,4-dioxo-1(2H)-pyrimidinecarboxamide

This compound was prepared by the similar procedure as in the case of Example 1, except for treatment with 2-[[3-(butyldimethylsilyl)propyl]thio]ethyl amine (11.7 g, 50.0 mmol) instead of 2-[[3-trimethylsilyl]propyl]thio]ethyl amine. The crude material was recrystallized from ethyl ether to yield the desired compound (18.4 g, 94.5%).

Melting point: 123—124°C

Elementary analysis: $C_{16}H_{28}FN_3O_3SSi$

Cal.: C, 49.33; H, 7.24; N, 10.79

Found: C, 49.50; H, 7.44; N, 10.72

IR ($\nu_{max}^{KBr}$) $cm^{-1}$:

3330 ($\nu_{NH}$), 2850, 2830 ($\nu_{CH}$), 1735 ($\nu_{C=O}$), 1680 ($\nu_{CONH}$)

$^1H$—NMR ($CDCl_3$) δ ppm;

— 0.03 (6H, s, $(CH_3)_2Si$—)

0.45—0.82 (2H, m, ⪢$SiCH_2$—)

0.89 (9H, s, —$C(CH_3)_3$)

1.32—1.92 (2H, m, ⪢$SiCH_2CH_2$—)

2.45—2.90 (4H, m, —$CH_2SCH_2$—)

3.62 (2H, q, J=6.0Hz, —$NHCH_2$—)

8.45 (1H, d, J=7.0Hz, $C_6$—H)

9.22 (2H, br., 2 × NH)

## Example 14

N-[3-[[3-(t-Butyldimethylsilyl)propyl]thio]propyl]-5-fluoro-3,4-dihydro-2,4-dioxo-1(2H)-pyrimidinecarboxamide

This compound was prepared by the similar procedure as in the case of Example 1, except for treatment with 3-[[3-(t-butyl-dimethylsilyl)propyl]thio]propyl amine (12.4 g, 50.0 mmol) instead of 2-[[3-(trimethylsilyl)propyl]thio]ethyl amine. The crude material was recrystallized from ethyl ether-n-hexane to yield the desired compound (18.4 g, 91.4%).

Melting point: 102—103°C

Elementary analysis: $C_{17}H_{30}FN_3O_3SSi$

Cal.: C, 50.59; H, 7.49; N, 10.41

Found: C, 50.34; H, 7.66; N, 10.45

IR ($\nu_{max}^{KBr}$) $cm^{-1}$:

3350 ($\nu_{NH}$), 3080, 2960, 2940, 2860 ($\nu_{CH}$), 1730 ($\nu_{C=O}$), 1670 ($\nu_{CONH}$)

$^1H$—NMR ($CDCl_3$) δ ppm:

— 0.90 (6H, s, —$Si(CH_3)_2$)

0.38—0.75 (2H, m, —$CH_2Si$⪡)

0.85 (9H, s, —$C(CH_3)_3$)

1.25—2.18 (4H, m, ⪢$SiCH_2CH_2CH_2SCH_2CH_2$—)

2.35—2.75 (4H, m, ⪢$SiCH_2CH_2CH_2SCH_2CH_2$—)

3.31—3.75 (2H, m, —$CH_2NH$—)

8.50 (1H, d, J=7.0Hz, $C_6$—H)

9.11 (1H, br., NH)

MS (EI/DI) m/z:

216 (base peak)

## Example 15

N-[3-[[3-(Butyldimethylsilyl)propyl]thio]propyl]-5-fluoro-3,4-dihydro-2,4-dioxo-1(2H)-pyrimidinecarboxamide

This compound was prepared by the similar procedure as in the case of Example 1, except for treatment with 3-[[3-(butyldimethylsilyl)propyl]thio]propyl amine (12.4 g, 50.0 mmol) instead of 2-[[3-(trimethylsilyl)propyl]thio]ethyl amine. The crude material was recrystallized from ethyl ether-n-hexane to yield the desired compound (19.3 g, 95.7%).

Melting point: 71—72°C

Elementary analysis: $C_{17}H_{30}FN_3O_3SSi$

Cal.: C, 50.59; H, 7.49; N, 10.41

Found: C, 51.10; H, 7.88; N, 10.44

$^1H$—NMR ($CDCl_3$) δ ppm:

— 0.07 (6H, s, —$Si(CH_3)_2$)

0.31—2.21 (15H, m, —$CH_2CH_2Si(CH_2)_3CH_3$, —$SCH_2CH_2CH_2NH$—)

2.38—2.75 (4H, m, ⪢$SiCH_2CH_2CH_2SCH_2$—)

3.33—3.71 (2H, m, —$CH_2NH$—)

8.46 (1H, d, J=7.0Hz, $C_6$—H)

9.06 (1H, br., NH)

MS (EI/DI) m/z:

258, 216 (base peak)

Example 16

N-[3-[[3-(Dimethylphenylsilyl)propyl]thio]propyl]-5-fluoro-3,4-dihydro-2,4-dioxo-1(2H)-pyrimidinecarboxamide

This compound was prepared by the similar procedure as in the case of Example 1, except for treatment with 3[[3-(dimethylphenylsilyl)propyl]thio]propyl amine (13.4 g, 50.0 mmol) instead of 2-[[3-(trimethyl-silyl)propyl]thio]ethyl amine. The crude material was recrystallized from ethyl ether-n-hexane to yield the desired compound (19.8 g, 93.2%).

Melting point: 51—52°C

Elementary analysis: $C_{19}H_{26}FN_3O_3SSi$

Cal.: C, 53.88; H, 6.19; N, 9.92

Found: C, 54.57; H, 6.49; N, 9.79

IR ($\nu_{max}^{KBr}$) $cm^{-1}$:

3350 ($\nu_{NH}$), 3080, 2960, 2930 ($\nu_{CH}$), 1735 ($\nu_{C=O}$), 1690 ($\nu_{CONH}$)

$^1H$—NMR ($CDCl_3$) δ ppm;

0.22 (6H, s, $Ph(CH_3)_2Si$—)

0.65—1.05 (2H, m, $Ph(CH_3)_2SiCH_2$—)

1.22—2.08 (4H, m, $Ph(CH_3)_2SiCH_2CH_2CH_2SCH_2CH_2$—)

2.35—2.72 (4H, m, $Ph(CH_3)_2SiCH_2CH_2CH_2SCH_2$—)

3.28—3.72 (2H, m, —$CH_2NH$—)

7.18—7.62 (5H, m, $Ph(CH_3)_2Si$—)

8.44 (1H, d, J=7.0Hz, $C_6$—H)

9.05 (2H, br., 2 × NH)

MS (EI/DI) m/z:

278, 137 (base peak)

Example 17

N-[3-[[2-(Butyldimethylsilyl)ethyl]thio]propyl]-5-fluoro-3,4-dihydro-2,4-dioxo-1(2H)-pyrimidinecarboxamide

This compound was prepared by the similar procedure as in the case of Example 1, except for treatment with 3-[[2-(butyldimethylsilyl)ethyl]thio]propyl amine (11.7 g, 50.0 mmol) instead of 2-[[3-(trimethylsilyl)propyl]thio]ethyl amine. The crude material was recrystallized from n-propanol to yield the desired compound (11.9 g, 61.2%).

Melting point: 83.5—84°C

Elementary analysis: $C_{16}H_{28}FN_3O_3SSi$

Cal.: C, 49.33; H, 7.24; N, 10.79

Found: C, 49.36; H, 7.41; N, 10.87

IR ($\nu_{max}^{KBr}$) $cm^{-1}$:

3340 ($\nu_{NH}$), 3090, 2930 ($\nu_{CH}$), 1740 ($\nu_{C=O}$), 1690 ($\nu_{CONH}$)

$^1H$—NMR ($CDCl_3$) δ ppm:

— 0.08 (6H, s, —$Si(CH_3)_2$)

0.2—1.4 (11H, m, —$CH_2SiCH_2CH_2CH_2CH_3$)

1.87 (2H, q, J=7.0Hz, —$NCH_2CH_2CH_2S$—)

2.3—2.9 (4H, m, —$CH_2SCH_2$)

3.47 (2H, q, J=7.0Hz, —$NCH_2$—)

8.48 (1H, d, J=7.0Hz, $C_6$—H)

8.9—9.3 (2H, br., NH × 2)

FAB—MS m/z:

390[$(M+1)^+$]

Example 18

N-[3-[[2-(Dimethylphenylsilyl)ethyl]thio]propyl]-5-fluoro-3,4-dihydro-2,4-dioxo-1(2H)-pyrimidinecarboxamide

This compound was prepared by the similar procedure as in the case of Example 1, except for treatment with 3-[[2-(dimethylphenylsilyl)ethyl]thio]propyl amine (12.7 g, 50.0 mmol) instead of 2-[[3-(trimethylsilyl)propyl]thio]ethyl amine. The crude material was recrystallized from ethyl ether to yield the desired compound (15.1 g, 73.7%).

Melting point: 93.5—94°C

Elementary analysis: $C_{18}H_{24}FN_3O_3SSi$

Cal.: C, 52.79; H, 5.91; N, 10.26

Found: C, 52.88; H, 6.20; N, 10.37

IR ($\nu_{max}^{KBr}$) $cm^{-1}$:

3320 ($\nu_{NH}$), 3060, 2960, 2840 ($\nu_{CH}$), 1745 ($\nu_{C=O}$), 1690 ($\nu_{CONH}$)

$^1H$—NMR ($CDCl_3$) δ ppm:

0.32 (6H, s, —$Si(CH_3)_2$)

0.9—1.3 (2H, m, —$CH_2Si$<)

1.90 (2H, q, J=7.0Hz, —$NCH_2CH_2CH_2S$—)

2.4—2.9 (4H, m, —$CH_2SCH_2$)

3.55 (2H, q, J=7.0Hz, —$NCH_2$—)

7.3—7.8 (5H, m, Ar—H)

8.57 (1H, d, J=7.0Hz, $C_6$—H)

8.9—9.4 (2H, br., NH × 2)

FAB—MS m/z:

410[$(M+1)^+$]

Pharmacological Test Example 1

(Suppression of KB cell proliferation in vitro)

In a cultivation device (5% $CO_2$, 37°C), KB cells (1 × $10^4$ cells) were cultivated for 24 hours in a culture tube with 1 ml of Eagle culture medium with 10% by wt of ox serum added, the used culture medium was discarded and replaced by a fresh culture medium (0.9 m), and then 0.1 ml of a similar culture fluid containing each test compound prepared by the process as described in the Examples was added to cultivate the same for further 3 days. Thereafter, living cells were dyed with Tripan Blue and measured by a dye-exclusion method.

A control was treated in a manner similar to the above, except that a culture fluid containing no compound of this invention was added.

50% inhibition density ($IC_{50}$) to the tumor cell proliferation was decided through the tests between the test group and the control group to obtain results as shown in following Table 1.

Table 1

| Compound (Example) | KB $IC_{50}$, μg/ml |
|---|---|
| 1 | 20 |
| 2 | 13 |
| 3 | 12 |
| 4 | 20 |
| 5 | 4 |
| 6 | 12 |
| 7 | 4 |
| 8 | 7 |
| 9 | 15 |
| 10 | 10 |
| 11 | 13 |
| 12 | 15 |
| 13 | 13 |

Pharmacological Test Example 2

(Anti-tumor action to L—1210 leukemia)

L—1210 leukemia cell (1 × $10^5$ cells) was transplanted in a abdominal cavity of $BDF_1$ female mice (20—21 g, age of about 5 weeks), and each test compound was orally dosed in 250 mg/kg or 500 mg/kg at 1st, 5th and 9th day after the transplantation to check days of life of the mice. Results are shown in following Table 2.

$$\text{ILS (\%)} = \frac{T-C}{C} \times 100$$

T: Days to death on testing group,
C: Days to death on control group

Table 2

| Compound (Example) | Dosage/day x 3 (mg/kg) | L-1210 ILS (%) | L-1210 Judgement* |
|---|---|---|---|
| 1 | 250 | 28 | + |
| | 500 | 98 | ++ |
| 2 | 250 | 19 | - |
| | 500 | 96 | ++ |
| 3 | 250 | 38 | + |
| | 500 | 82 | ++ |
| 4 | 250 | 12 | - |
| | 500 | 38 | + |
| 5 | 250 | 51 | + |
| | 500 | 106 | +++ |
| 6 | 250 | 35 | + |
| | 500 | 73 | ++ |
| 7 | 250 | 11 | - |
| | 500 | 11 | - |
| 8 | 250 | 27 | + |
| | 500 | 70 | ++ |
| 9 | 250 | 45 | + |
| | 500 | 80 | ++ |

Table 2 (continued)

| Compound (Example) | Dosage/day x 3 (mg/kg) | L-1210 | |
|---|---|---|---|
| | | ILS (%) | Judgement* |
| 10 | 250 | 49 | + |
| | 500 | 111 | +++ |
| 11 | 250 | 18 | - |
| | 500 | 81 | ++ |
| 12 | 250 | 24 | - |
| | 500 | 70 | ++ |
| 13 | 250 | 10 | - |
| | 500 | 67 | ++ |
| 14 | 250 | 19 | - |
| | 500 | 68 | ++ |
| 15 | 250 | 26 | + |
| | 500 | 62 | ++ |
| 16 | 250 | 23 | - |
| | 500 | 86 | ++ |

* - : $25 > ILS$

+ : $25 \leqq ILS < 50$

++ : $50 \leqq ILS < 100$

+++ : $100 \leqq ILS$

Pharmacological Test Example 3

(Anti-tumor action to Colon-38 carcinoma)

0.02 ml of 10(W/V)% cell suspension prepared from a solid cancer cell (Colon-38 carcinoma) were transplanted under skin of $BDF_1$ mice (20—21 g, age of about 5 weeks), and each test compound was orally dosed to the mice at 1st, 5th and 9th day after the transplantation. Major and minor axes of the cancerous tumor were measured at 11th, 14th, 21st, 25th and 28th day after the transplantation to calculate the volume of the tumor in accordance with the following equation:

$$\frac{1}{2} L \times W^2$$

L: major axis (mm)
W: minor axis (mm)

At 28th day from the transplantation, the mice were killed and each of the cancerous tumors was

extracted to measure its weight and to determine an inhibition ratio in comparison with a non-treated control group.

Results are shown in the following Table 3.

Table 3

| Compound (Example) | Dosage/day x 3 (mg/kg) | colon-38 Inhibition ratio (%) |
|---|---|---|
| 1 | 278 | 95.2 |
| 2 | 300 | 71.9 |
| 3 | 315 | 82.1 |
| 4 | 345 | 80.0 |
| 5 | 289 | 89.8 |
| 6 | 300 | 81.2 |
| 7 | 366 | 32.2 |
| 8 | 278 | 78.0 |
| 9 | 338 | 98.4 |
| 10 | 289 | 89.5 |
| 11 | 350 | 90.3 |
| 12 | 338 | 97.3 |
| 13 | 311 | 81.0 |
| 14 | 323 | 90.1 |
| 15 | 323 | 92.3 |
| 16 | 339 | 97.9 |
| 17 | 312 | 97.4 |
| 18 | 328 | 71.3 |

Pharmacological Test Example 4

(Anti-tumor action to MM—46 adenocarcinoma)

MM—46 adenocarcinoma (1 × $10^6$ cells) was transplanted under skin of C3H/He female mice (age of about 5 weeks), and each test compound was orally dosed at 1st, 5th and 9th day after the transplantation to check days of life of the mice.

Results were shown in following Table 4.

In the Table, T: days to death on testing group, and
C: days to death on control group.

Table 4

| Compound (Example) | Dosage/day x 3 (mg/kg) | MM-46 adenocarcinoma T/C x 100 (%) |
|---|---|---|
| 1 | 250 | 181 |
| 3 | 250 | 205 |
| 5 | 250 | 162 |

Pharmacological Test Example 5

(Anti-tumor action to B—16 melanoma)

B—16 melanoma (1 × $10^5$ cells) was transplanted under skin of $BDF_1$ female mice (age of about 5 weeks), and each of testing compounds was orally dosed at 1st, 5th and 9th day after the transplantation. At 21st day after the transplantation, the mice were killed to measure a weight of the cancerous tumor and to finally determine an inhibition ratio.

Results are shown in the following Table 5.

Table 5

| Compound (Example) | Dosage/day x 3 mg/kg | B-16 melanoma Inhibition ratio (%) |
|---|---|---|
| 1 | 250 | 73.8 |
| | 125 | 25.8 |
| 2 | 300 | 47.2 |
| 3 | 300 | 62.0 |
| 5 | 300 | 79.7 |
| 6 | 300 | 53.5 |
| 8 | 250 | 4.3 |
| 9 | 250 | 19.4 |
| | 125 | 17.6 |
| 10 | 250 | 15.2 |
| | 125 | 38.1 |
| 11 | 250 | 45.7 |
| | 125 | 32.8 |
| 12 | 250 | 37.9 |
| | 125 | 34.2 |
| 13 | 250 | 43.1 |
| | 125 | 38.0 |
| 14 | 250 | 46.3 |
| | 125 | 55.2 |
| 15 | 250 | 88.5 |
| | 125 | 69.5 |
| 16 | 250 | 70.5 |
| | 125 | 82.2 |
| 17 | 400 | 34.8 |
| | 200 | 16.3 |
| | 100 | 54.3 |
| 18 | 400 | 29.6 |
| | 200 | 46.7 |
| | 100 | 55.2 |

Pharmacological Test Example 6

(Anti-tumor action on Lewis lung carcinoma)

Lewis lung carcinoma ($1 \times 10^5$ cells) was transplanted under the skin of the right ear of $BDF_1$ mice (age of about 5 weeks), and each test compound was orally dosed 10 times from 1st to 10th day after the transplantation. At the 21st day after the transplantation, the mice were killed to measure the weight of the primary tumor and to extract a lung. The lung was fixed in a conventional manner to count the number of metastases on cancerous cells by the use of a stereomicroscope having a low magnification. The anti-tumor action of each compound was expressed as the inhibition in weight of the primary tumor and inhibition of the metastasis.

Results are shown in the following Table 6.

Table 6

| Compound (Example) | Dosage/day x 10 (mg/kg) | Inhibition Ratio weight LLC (%) | metastasis (%) |
|---|---|---|---|
| 1 | 13 | 0.0 | 50.9 |
| | 13 + Amputation | 33.2 | (26.6) |
| | 26 + Amputation | 59.8 | (>63.0) |
| | 26 | 51.1 | 79.2 |
| | 52 | 31.1 | 95.7 |
| 2 | 14 | 0.0 | 45.2 |
| 3 | 29.5 | 20.7 | 57.5 |
| | 59 | 0.0 | 95.1 |
| 4 | 32 | 4.6 | 76.2 |
| | 64 | 18.0 | 97.5 |
| 5 | 13.5 | 0.0 | 19.3 |
| | 13.5 + Amputation | 34.7 | (15.4) |
| | 27 + Amputation | 35.8 | (>39.5) |
| | 27 | 7.1 | 78.4 |
| | 54 | 34.9 | 83.0 |
| 6 | 13.5 + Amputation | 37.3 | (18.3) |
| | 27 + Amputation | 30.9 | (23.1) |
| | 27 | 39.6 | 92.6 |
| | 54 | 38.0 | 95.1 |
| 7 | 34 | 31.0 | 67.1 |
| | 68 | 25.0 | 87.7 |
| 8 | 13 | 0.0 | 53.0 |
| | 13 + Amputation | 33.3 | (18.4) |
| | 26 | 26.2 | 92.5 |
| | 26 + Amputation | 64.9 | (33.6) |

Pharmacological Test Example 7

(Acute toxicity)

Each test compound suspended in 0.1% of P—1570 was orally and forcedly dosed through a gastric probe to CrJ/CDI female mice (21 to 22 g, age about 5 weeks). An identification number was given to each mouse, and a weighing and observation on general behaviour were carried out just before the dosage and each day thereafter. Each dead mouse was subjected without delay to an autopsy, more particularly in detail on enterona. All the living mice were killed at the 21st day from the dosage to carry out an autopsy, more particularly on the entrails.

An $LD_{50}$ was calculated in accordance with Leed and Munch's method.

A similar test was carried out on each control of 1-[2-[(2-tri-methylsilylethyl)thio]ethylcarbamoyl]-5-fluorouracil and 1-hexyl-carbamoyl-5-fluorouracil (HCFU) which are known anti-tumor agents. On the former compound, a spasm was observed in some mice and an autopsy of these mice showed an ulcer and loose faeces, none of which was seen in an autopsy on mice dosed with a compound according to the invention.

P—1570 is an edible non-ion surface active agent marketed by Ryoto Co., Ltd. of Tokyo, Japan. This comprises a mixture of esters between sucrose and fatty acids and has following physico-chemical and other data.

1. Fatty acid composition:
   Stearic acid about 30%
   Palmitic acid about 70%
2. Ester composition:
   mono-ester about 70%
   di and tri-esters about 30%
3. Softening point:
   48—55°C
4. HLB;
   14—15

Results are shown in the following Table 7.

Table 7

| Compounds | $LD_{50}$ (mg/kg) |
|---|---|
| Examples | |
| 1 | 1575 |
| 2 | 1545 |
| 3 | 1500 |
| 4 | 1580 |
| 5 | 1380 |
| 6 | 1385 |
| 7 | 1400 |
| 8 | 1355 |
| 9 | 2250 |
| 10 | 2170 |
| 11 | 2120 |
| 12 | 1820 |
| 13 | 1380 |
| 14 | 1800 |
| 15 | 1850 |
| 16 | 1800 |
| Controls | |
| 1-[2-[(2-trimethylsilylethyl)thio]-ethylcarbamoyl]-5-fluorouracil | 1095 |
| HCFU | 1250 |

Prescription Example 1 (Tablet)

Following components were mixed to prepare tablets in a conventional manner.

| | |
|---|---|
| Compound of Example 1 | 100 (mg) |
| Crystalline cellulose | 20 |
| Lactose | 41 |
| Corn starch | 30 |
| Hydroxypropylcellulose | 6 |
| Magnesium stearate | 3 |
| | 200 mg/tablet |

Prescriptional Example 2 (Capsule)

Following components were mixed and charged to capsules in a conventional manner to prepare capsuled pharmaceutical agents.

| | |
|---|---|
| Compound of Example 5 | 200 (mg) |
| Crystalline cellulose | 50 |
| Silicic anhydride | 2 |
| Magnesium stearate | 3 |
| | 255 mg/capsule |

Prescriptional Example 3 (Granule)

Following components were mixed and packed in a conventional manner to prepare granular pharmaceutical agents.

| | |
|---|---|
| Compound of Example 8 | 500 (mg) |
| Lactose | 323 |
| Corn starch | 150 |
| Polyvinylpyrrolidone | 25 |
| Silicic anhydride | 2 |
| | 1000 mg/package |

Prescriptional Example 4 (Suppository)

Following components were mixed to prepare suppositories in a conventional manner.

| | |
|---|---|
| Compound of Example 10 | 300 (mg) |
| Witepsol W—35 | 1700 |
| | 2000 mg/suppository |

Witep-Sol W—35 has widely been known as a basic material for preparing a suppository and is available in various countries. This comprises a mixture of mono, di and tri-glycerides of saturated fatty acids from lauric acid ($C_{11}$) to stearic acid ($C_{17}$). Physico-chemical data of the specific Witepsol used are given as follows.

1. Melting point: 33.5—35.5°C
2. Solidification point: 27—32°C
3. Iodine colour index: below 3
4. Acid value: below 0.3
5. Saponification value: 225—235
6. Iodine value: below 3
7. Hydroxyl value: 40—50
8. Unsaponifiable matter: below 0.3
9. Mean molecular weight: about 615

## Claims

1. An organo-silicone compound represented by the formula

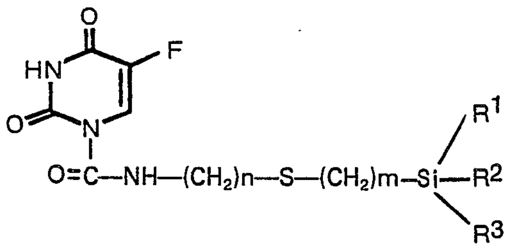

wherein $R^1$, $R^2$ and $R^3$ are each an alkyl group of 1 to 10 carbon atoms or a phenyl radical, and *m* and *n* are each an integer of 2 or 3, but are not both 2 when each of $R^1$, $R^2$ and $R^3$ represents a methyl radical.

2. A compound as claimed in Claim 1, wherein $R^1$ and $R^2$ are each methyl, ethyl or phenyl.

3. A compound as claimed in Claim 1 or 2, wherein $R^3$ is methyl, ethyl, n-butyl, t-butyl, n-octyl, isopropyl or phenyl.

4. A process for the preparation of an organo-silicone compound as claimed in any preceding claim, characterized in that a compound represented by the formula

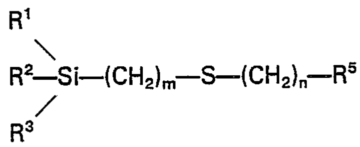

(II)

wherein $R^1$, $R^2$, $R^3$, *m* and *n* are as defined in Claim 1, and $R^5$ is the radical $-N=C=O$, $-NH_2$ or $-NH-CO-Cl$, is reacted with a compound represented by the formula:

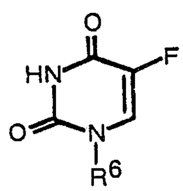

(III)

wherein $R^6$ is hydrogen, an alkali metal or the radical $-COCl$.

5. A process as claimed in Claim 4, wherein a compound represented by the formula (II) where $R^5$ is $-NH_2$ is reacted with a 1-chloroformyl-5-substituted uracil represented by the formula (III) where $R^6$ is $-COCl$.

6. A process as claimed in Claim 4, wherein a compound represented by the formula (II) where $R^5$ is $-NR^4-CO-Cl$ is reacted with a 5-substituted uracil represented by the formula (III) where $R^6$ is a hydrogen or alkali metal atom.

7. A process as claimed in Claim 4, wherein an isocyanate represented by the formula (II) where $R^5$ is $-N=C=O$ is reacted with a 5-substituted uracil represented by the formula (III) where $R^6$ is a hydrogen atom.

8. An anti-tumor pharmaceutical composition which comprises as an effective component at least one organo-silicone compound as claimed in any of Claims 1 to 3.

## Patentansprüche

1. Organo-Silicium-Verbindung der Formel

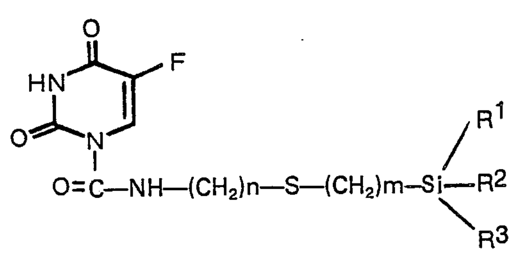

worin $R^1$, $R^2$ und $R^3$ je eine Alkylgruppe mit 1 bis 10 Kohlenstoffatomen oder ein Phenylrest sind und *m* und

*n* je die ganze Zahl 2 oder 3 sind, jedoch nicht beide 2 sind, wenn alle Reste $R^1$, $R^2$ und $R^3$ einen Methylrest bedeuten.

2. Verbindung gemäss Anspruch 1, worin $R^1$ und $R^2$ je Methyl, Ethyl oder Phenyl sind.

3. Verbindung gemäss Anspruch 1 oder 2, worin $R^3$ Methyl, Ethyl, n-Butyl, t-Butyl, n-Octyl, Isopropyl oder Phenyl sind.

4. Verfahren für die Herstellung einer Organo-Silicium-Verbindung gemäss einem der vorhergehenden Ansprüche, dadurch gekennzeichnet, dass eine Verbindung der Formel

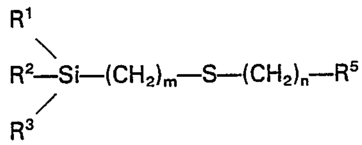

(II)

worin $R^1$, $R^2$, $R^3$, *m* und *n* wie im Anspruch 1 definiert sind und $R^5$ der Rest $-N{=}C{=}O$, $-NH_2$ oder $-NH-CO-Cl$ ist, mit einer Verbindung der Formel

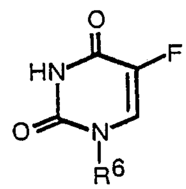

(III)

worin $R^6$ Wasserstoff, Alkalimetall oder den Rest $-COCl$ bedeutet, umgesetzt wird.

5. Verfahren gemäss Anspruch 4, worin eine Verbindung der Formel (II), worin $R^5$ $-NH_2$ ist, mit einem 1-Chlorformyl-5-substituierten Uracil der Formel (III), worin $R^6$ $-COCl$ ist, umgesetzt wird.

6. Verfahren gemäss Anspruch 4, worin eine Verbindung der Formel (II), worin $R^5$ $-NR^4-CO-Cl$ ist, mit einem 5-substituierten Uracil der Formel (III), worin $R^6$ Wasserstoff oder ein Alkalimetallatom ist, umgesetzt wird.

7. Verfahren gemäss Anspruch 4, worin ein Isocyanat der Formel (II), worin $R^5$ $-N{=}C{=}O$ ist, mit einem 5-substituierten Uracil der Formel (III), worin $R^6$ ein Wasserstoffatom ist, umgesetzt wird.

8. Pharmazeutische Antitumor-Zusammensetzung, welche als wirksame Komponente mindestens eine Organo-Silicium-Verbindung gemäss einem der Ansprüche 1 bis 3 enthält.

**Revendications**

1. Composé organo-silicone représenté par la formule

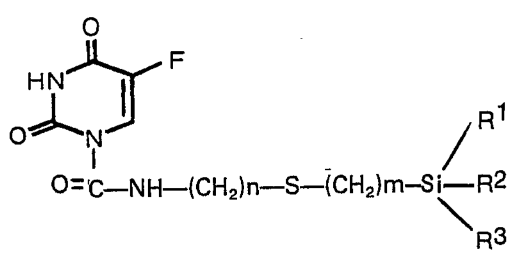

dans laquelle $R^1$, $R^2$ et $R^3$ sont chacun un groupe alkyle de 1 à 10 atomes de carbone ou un radical phényle, et *m* et *n* sont chacun le nombre entier 2 ou 3, mais ne sont pas tous deux 2 lorsque $R^1$, $R^2$ et $R^3$ représentent tous un radical méthyle.

2. Composé selon la revendication 1, dans lequel $R^1$ et $R^2$ sont chacun un groupe méthyle, éthyle ou phényle.

3. Composé selon la revendication 1 ou 2, dans lequel $R^3$ est un groupe méthyle, éthyle, n-butyle, t-butyle, n-octyle, isopropyle ou phényle.

4. Procédé de préparation d'un composé organo-silicone selon l'une quelconque des revendications précédentes, caractérisé en ce qu'on fait réagir un composé représenté par la formule

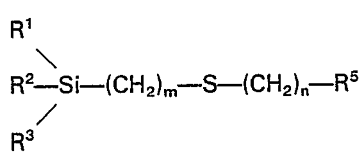

(II)

dans laquelle $R^1$, $R^2$, $R^3$, *m* et *n* sont tels que définis dans la revendication 1 et $R^5$ est le radical —N=C=O, —$NH_2$ ou —NH—CO—Cl, avec un composé représenté par la formule:

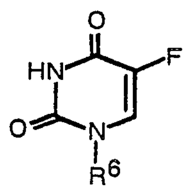

(III)

dans laquelle $R^6$ est un atome d'hydrogène, d'un métal alcalin ou le radical —COCl.

5. Procédé selon la revendication 4, dans lequel on fait réagir un composé représenté par la formule (II) où $R^5$ est —$NH_2$ avec un 1-chloroformyl-uracil-substitué-5 représenté par la formule (III) où $R^6$ est —COCl.

6. Procédé selon la revendication 4, dans lequel on fait réagir un composé représenté par la formule (II) où $R^5$ est —$NR^4$—CO—Cl avec un uracile substitué-5 représenté par la formule (III) où $R^6$ est un atome d'hydrogène ou de métal alcalin.

7. Procéde selon la revendication 4, dans lequel on fait réagir un isocyanate représenté par la formule (II) où $R^5$ est —N=C=O avec un uracile substitué-5 représenté par la formule (III) où $R^6$ est un atome d'hydrogène.

8. Composition pharmaceutique anti-tumorale comprenant comme composant efficace au moins un composé organo-silicone selon l'une quelconque des revendications 1 à 3.